# EUROPEAN PATENT SPECIFICATION

(11) **EP 2 758 003 B1**
(45) Date of publication and mention of the grant of the patent: **11.11.2020**
(21) Application number: 11776023.1
(22) Date of filing: 19.09.2011
(51) Int. Cl.: A61F 2/36, A61F 2/30

(54) **MODULAR SPACER DEVICE FOR THE TREATMENT OF INFECTIONS OF THE PROSTHESIS OF THE HUMAN LIMBS**
MODULARER ABSTANDSHALTER ZUR BEHANDLUNG VON INFEKTIONEN DER PROTHESE MENSCHLICHER EXTREMITÄTEN
DISPOSITIF D'ÉCARTEMENT MODULAIRE POUR LE TRAITEMENT D'INFECTIONS DE LA PROTHÈSE DES MEMBRES HUMAINS

(43) Date of publication of application: 30.07.2014
(73) Proprietor: Tecres S.P.A., 37066 Sommacampagna (VR) (IT)
(72) Inventor: FACCIOLI, Giovanni, 37066 Sommacampagna (Verona) (IT); SOFFIATTI, Renzo, 37066 Sommacampagna (Verona) (IT)
(74) Representative: Feltrinelli, Secondo Andrea
(86) International application number: PCT/IB2011/054094
(87) International publication number: WO 2013/041906

(56) References cited:
- EP-A2- 0 768 066
- WO-A1-91/18559
- WO-A1-2010/015877
- DE-A1- 19 952 918
- DE-A1-102008 030 260
- US-A- 4 170 794
- US-A- 4 225 981
- US-A1- 2001 051 831

## Description

### TECHNICAL FIELD OF THE INVENTION

The present invention regards a spacer device for the two-stage treatment of arthroprosthesis infections, for example hip prostheses, humeral prostheses, knee prostheses, ankle prostheses, etcetera.

### STATE OF THE PRIOR ART

Arthroprosthesis infections are among the most serious causes of failure of an arthroprosthesis. With specific reference to hip arthroprostheses, such events occur quite often with a percentage variable between 0.5% and the 6% of the cases. The percentage of negative events increases in case of re-implantation or in presence of risk factors such as previous interventions, local haematoma, intercurrent infectious diseases, local or general bone diseases, immunosuppression, etcetera.

A method for curing the infection, defined two-stage treatment, provides for a first stage of removing the infected arthroprosthesis, the possibilities of success of the conservative antibiotic treatment are actually quite limited, and a second stage of a new re-implant of arthroprosthesis upon clearing the tissues of the patient in terms of infection.

In order to maintain the space required for the new re-implantation of arthroprosthesis and cure the infection, the applicant developed special arthroprostheses for temporary use, also referred to as spacers or temporary spacers, which release pharmaceutical and/or therapeutic products and allow having the articular mobility.

The aforementioned spacers are disclosed by the Italian patent n° IT-1278853 and European patent n° EP-1274374 on behalf of the applicant and incorporated herein for reference.

The International patent application WO-2007/099232 discloses a temporary spacer comprising a semispherical head to be inserted in the corresponding articulation separable and connectable to a stem to be inserted in the bone bed remaining from a previous implant. The spacer illustrated in WO-2007/099232 allows combining a stem with semispherical heads of different sizes so as to best adapt to the anatomy of the articulation of the patient.

The International application n. WO2010/015877, in the name of the same Applicant, discloses a spacer device for the two-step treatment of prosthesis infections, that is made from a biologically compatible and porous material designed to allow the possibility of adding pharmaceutical products, active and/or therapeutic ingredients. The spacer device comprises a first portion designed to be fixed to a corresponding bone bed remaining from a previous implant, and a second portion designed to be inserted in a corresponding articular area of the patient, the first portion and the second portion being attached by adjustable connecting means.

The adjustable connecting means comprise a screw/nut screw connection.

There arises the need of providing a solution alternative to the known ones, in particular more efficient, safe and inexpensive as regards the mutual locking between the stem and the head.

### OBJECTS OF THE INVENTION

An object of the present invention is to improve the prior art.

Another object of the present invention is to obtain a spacer device that can be easily adapted to the different sizes of the patients.

Still another object of the present invention is to obtain a spacer device that is more efficient, safe and inexpensive as regards the operation of mutual locking between the stem and the head.

A further object of the present invention is to provide a spacer device that allows maintaining the articular functionality reducing the recovery times for the patient.

Still another object of the present invention is to provide a spacer device capable of also bearing dynamic loads, at least for a given period of time, awaiting the final re-implant.

According to an aspect of the invention a spacer device according to claim 1 is provided.

### BRIEF DESCRIPTION OF THE DRAWINGS

Further characteristics and advantages of the invention will be more apparent from the description of some embodiments of the present invention, illustrated by way of example in the attached drawings wherein:
figure 1 is a lateral view, partly sectioned, of the spacer device according to the present invention;
figure 2 is a lateral view, partly sectioned, of a spacer device according to the invention constituted by the coupling of a normal stem and by a small-sized sphere, entirely fastened;
figure 3 is a lateral view, partly sectioned, of a spacer device according to the invention constituted by the coupling of a normal stem and by a small-sized sphere, entirely unfastened;
figure 4 is a lateral view, partly sectioned, of a spacer device according to the invention constituted by the coupling of a normal stem and by a medium-sized sphere, entirely fastened;
figure 5 is a lateral view, partly sectioned, of a spacer device according to the invention constituted by the coupling of a normal stem and by a medium-sized sphere, entirely unfastened;
figure 6 is a lateral view, partly sectioned, of a spacer device according to the invention constituted by the coupling of a normal stem and by a large-sized sphere, entirely fastened;
figure 7 is a lateral view, partly sectioned, of a spacer device according to the invention constituted by the coupling of a normal stem and by a large-sized sphere, entirely unfastened;
figures 8,9,10 are lateral views of different embodiments of the stem, normal-sized, of the spacer device according to the present invention;
figures 11,12,13 are lateral views of different embodiments of the stem, long-sized, of the spacer device according to the present invention;
figure 14 is a lateral view, partly sectioned of another embodiment of the spacer device according to the present invention;
figure 15 is a lateral sectioned view of the stem of the spacer device of figure 14.

### EMBODIMENTS OF THE INVENTION

With reference to the figures, a spacer device according to the present invention, in particular a spacer device for the two-stage treatment of infections of the prosthesis of the human limbs is indicated in its entirety with 1.

In the illustrated embodiment express reference shall be made to a spacer for the treatment and the replacement of hip arthroprosthesis, even though it should be observed that the present invention can be used also for treating other types of prostheses, for example, humeral prostheses, knee prostheses, ankle prostheses etcetera.

The device 1 according to the present invention is made of biologically compatible material, it can be porous and it is adapted to be added and/or it can be added with one or more pharmaceutical products, active and/or therapeutic ingredients adapted to be released in the tissues of the patient adjacent to the device.

The materials for the spacer device according to the present invention can be selected from among metals, metal alloys, organic metals, ceramic, glass, plastic. Specifically the plastic can be selected from among thermoplastic polymers, such as acrylic resins, including all copolymers and acrylic alloys, polyethylene, polypropylene thermo-formable through injection moulding or through blow moulding.

In accordance with the invention, the biologically compatible material with which the spacer device is made comprises polymethylmethacrylate.

The material the spacer device according to the present invention is made of may already comprise one or a plurality of first pharmaceutical products, active and/or therapeutic ingredients, for example antibiotics, and also being porous it can be further added, for example by impregnation, with one or more pharmaceutical products, active and/or therapeutic ingredients identical or different with respect to the first pharmaceutical products, active and/or -therapeutic ingredients.

In a further version of the invention, the spacer does not comprise pharmaceutical products, active and/or therapeutic ingredients and is added, for example by impregnation, with one or more pharmaceutical products, active and/or therapeutic ingredients when implanting in the patient.

Thus, at least three different types of spacer materials can be used in terms of the pharmaceutical and therapeutic products:
material already comprising one or a plurality of pharmaceutical products, active and/or therapeutic ingredients, without the possibility of adding other pharmaceutical and/or therapeutic products;
material already comprising one or a plurality of pharmaceutical products, active and/or therapeutic ingredients with the possibility of adding other pharmaceutical and/or therapeutic products, for example by impregnation when the material is porous;
material not comprising any pharmaceutical product, active and/or therapeutic ingredient, with the possibility of adding one or a plurality of pharmaceutical products, active and/or therapeutic ingredients, when implanting in the patient, for example by impregnation, when the material is porous.

The pharmaceutical products, active and/or therapeutic ingredients may comprise antibiotics, antiseptics, antimycotics, chemotherapeutic substances, for example gentamicin, vancomycin, etcetera, or other active ingredients.

According to the figures, the device 1 comprises a first portion 2 adapted to be fixed to a corresponding residue bone bed of a previous implant, a second portion 3 adapted to be inserted in a corresponding articular area of the patient.

The first portion 2 and the second portion 3 are connected through joining means, indicated in their entirety with 4, which are of the adjustable type.

In addition, they are provided with the locking means adapted to fix the position of the adjustable joining means 4, better described hereinafter.

In the embodiment versions of the figures, which regard the articulation of the hip, the first portion 2 comprises a stem 5 to be inserted in the proximal part of a femur.

The first portion 2 is provided with an inner reinforcement core, for example made of metal material or any other material having suitable mechanical characteristics.

The stem 5 is substantially frusto-conical shaped and also comprises a widened portion 6, proximal to the end 7 for connection to the second portion 3.

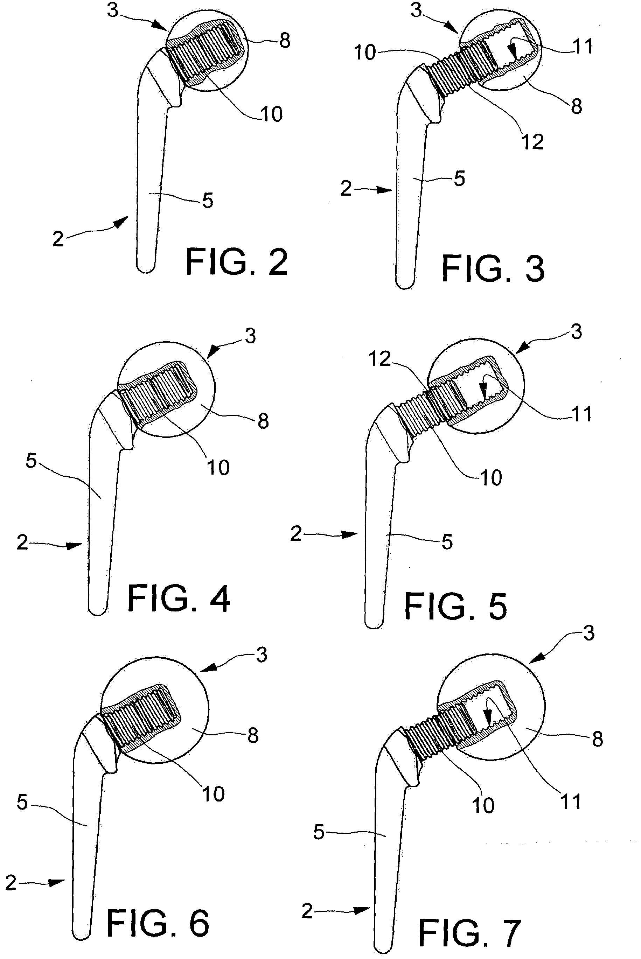

The second portion 3 of the device according to the invention comprises a substantially spherical head 8. Heads 8 with different dimensions, in particular with different diameter of the sphere so as to be adapted to the different sizes of the articular capsules of the patients can be provided for like those illustrated for example in figures 2-7.

For example, in figures 2,3 there is illustrated an embodiment of the spacer device comprising a head 8 that is small in size, respectively entirely fastened to or entirely unfastened from first portion 2.

In figures 4, 5 there is illustrated an embodiment of the spacer device comprising a head 8 that is medium-sized, respectively entirely fastened to or entirely unfastened from the first portion 2.

In figures 6, 7 there is illustrated an embodiment of the spacer device comprising a head 8 that is large-sized, respectively entirely fastened to or entirely unfastened from the first portion 2.

Figures 2-7, thus illustrate some of the possible combinations between the same first portion 2 of the spacer device with second portions 3 comprising variously sized heads 8, depending on the specific application needs, i.e. for an improved adaptation to the anatomy of the patient.

An important characteristic of the spacer device according to the present invention is constituted by the adjustable joining means 4 which, besides the function of connecting the first portion 2 and the second portion 3, also allow adjusting the mutual position between the first portion 2 and the second portion 3. In the embodiment of the figures, due to the adjustable joining means 4, it is possible to vary the length of the neck 9 of the device 1, once again for better adaptation to the anatomy of the patient on which the device is implanted.

According to an aspect of the present invention, the joining means 4 comprise mutual coupling surfaces 10,11 respectively provided in the first portion 2 and in the second portion 3.

The mutual coupling surfaces 10,11 are both coated with the biologically compatible material which comprises polymethylmethacrylate.

In other words, the mutual coupling surfaces 10,11 are coated with the same material with which the remaining parts of the first portion 2 and of the second portion 3 of the spacer device 1 are made.

The advantages of this solution will be clear hereinafter.

The aforementioned mutual coupling surfaces 10,11 comprise, more in detail, a screw 10 and a nut screw 11.

The screw 10 and the nut screw 11 are of the type having a threading with rounded profile.

For example, for better understanding, it should be observed that such profile is substantially similar to the combined profile of Edison type, or other similar profiles.

This solution allows obtaining a greater breaking strength and an improved capacity of the threads to be moulded in the production stage, and also other advantages to be better clarified in the description hereinafter.

Thus, there can also be provided profiles with greater pitch, so as to increase the coupling surfaces and thus increase the resistance of the coupling.

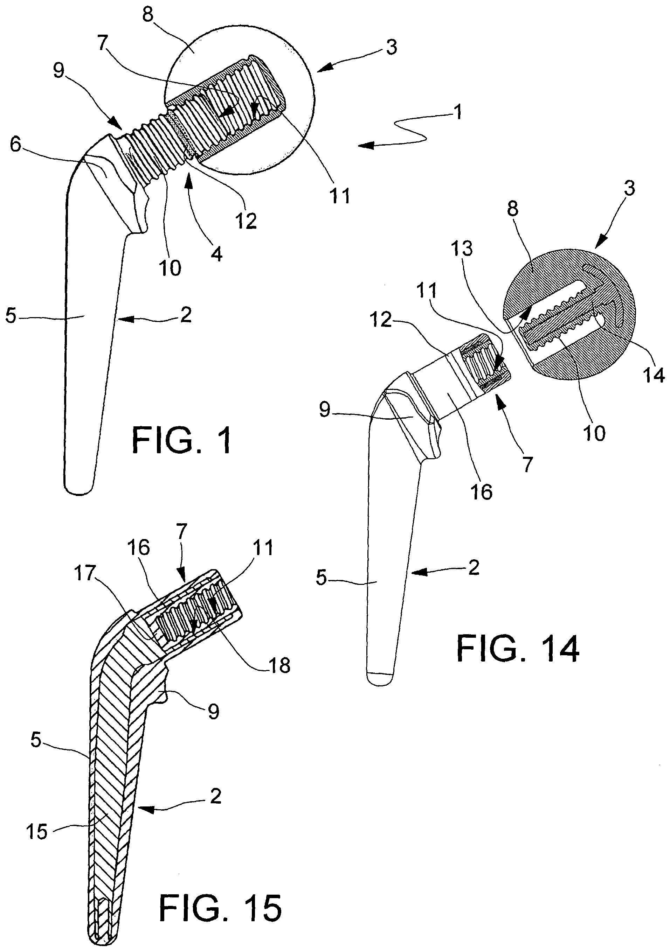

In the embodiments of figures 1-13, the screw 10 is provided in the first portion 2 of the device, i.e. it is obtained at the end 7 of the stem 5.

The nut screw 11 is instead provided in the second portion 3, i.e. it is comprised in the sphere 8. According to another aspect of the present invention, the first portion 2 of the spacer device comprises a visual indicator 12 of the position of complete unfastening of the second portion 3 with respect to the first portion 2.

Such visual indicator 12, in other words, allows identifying, along the thread of the screw 10, the limit position beyond which the second portion 3 should never be unfastened so as not to jeopardize the stability of the coupling between the two portions 2,3. More in particular, the visual indicator 12 comprises a band made along the outer surface of the screw 10. The band of the visual indicator 12 may be obtained, for example, using a special paint, or any other type of pigment, whose colour is clearly visible to the operator in any situation, or through other equivalent techniques.

As mentioned, figures 2-7 show various combinations of couplings between the same first portion 2 and different second portions 3, i.e. in particular between the same stem 5, normal-sized, and heads 8 of various sizes. In particular, figures 2,4,6 illustrate the device with the head 8 entirely fastened to the respective stem 5, while figures 3,5,7 illustrate the device with the head 8 entirely unfastened, i.e. unfastened until it moves to the visual indicator 12. Obviously, such positions represent the limit adjustment positions: the device can be adjusted in any intermediate position between the aforementioned limit positions.

It is thus clear that numerous combinations, regarding the sizes of the first portion 2, the sizes of the second portion 3 and the obtainable length of the neck 9 of the device 1 can be obtained, so as to meet any application requirement.

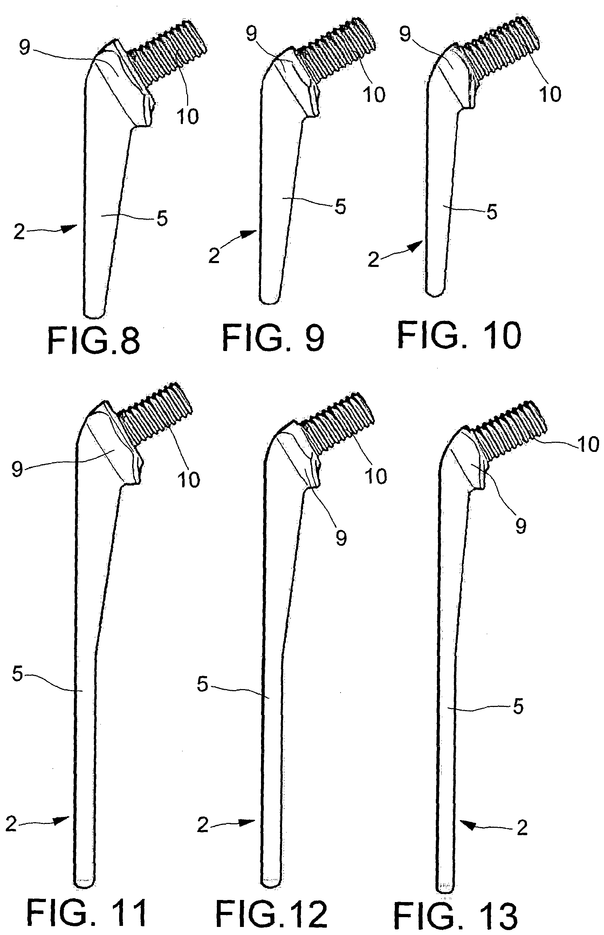

Figures 8-13 further illustrate different embodiments of the first portion 2 of the device according to the invention.

More in particular, figures 8-13 regard stems 5 of different shapes and sizes.

Figures 8-10 regard stems 5 of normal sizes provided with widened portions 6 of different dimensions, for a better adaptation to the anatomic characteristics of the patients.

In figures 11-13, instead, there are represented long-sized stems 5 provided with widened portions 6 of different sizes, still for a better adaptation to the anatomic characteristics of the patients.

According to an aspect of the present invention the means for locking the first portion 2 to the second portion 3, in the desired position required by the specific application, comprise portions of the aforementioned coupling surfaces 10,11 rigidly welded by applying a bonding component.

As clarified hereinafter, in a version of the invention, the bonding component, applied and left to act for a given period of time, provides for dissolving the surface layers of the biologically compatible material of the coupling surfaces 10,11 and then facilitating the subsequent mutual welding once brought to contact.

Regarding this, described hereinafter is a method which is not falling under the scope of the invention for the mutual locking of the first portion 2 to the second portion 3 of the spacer device according to the invention in the desired position, depending on the specific application requirements.

The method initially provides for a stage of distributing a predefined amount of bonding component on at least one of the coupling surfaces 10,11, which as mentioned are made of biologically compatible material comprising polymethylmethacrylate.

The bonding component can be of different type.

For example, acrylic bone cement, or a cyanoacrylate bonding agent, or even an organic solvent or a mixture of several components, such as chloroform, methyl methacrylate, ethyl acetate, dichloromethane and many others can be used.

Obviously, any component that is used should be stable and biocompatible.

Such stage of distributing the bonding component is performed, in particular, by filling the nut screw 11 of the second portion 3 with the bonding component.

The bonding component can be made available for example in a special vial, which is opened at the moment of use.

Subsequently there is provided a stage for applying a closure cap to the nut screw 11, so as not to disperse the bonding component.

The closure cap is small in size, in particular short, so as not to obstruct the first threads of the nut screw 11.

The two parts can be subsequently immediately coupled, but it is preferable to provide for a stage of waiting for a given period of time, for example about one minute, to allow the bonding agent to act and complete the effect thereof on polymethylmethacrylate.

Once such period of time has elapsed, the closure cap is removed and the coupling surfaces 10,11 - screw and nut screw - of the first portion 2 and of the second portion 3, are joined in the desired position.

More in detail, the head 8 is fastened to the screw 10 up to the desired position, so as to obtain a neck 9 of the desired length.

Lastly, there is provided a stage of waiting for a further predefined period of time to allow the mutual stable fixing of the coupling surfaces 10,11; in such stage the device should not be subjected to movements so as not to lose the mutual positioning of the first portion 2 and of the second portion 3.

Such predefined period of time can be, for example, about one hour or at least one hour.

Thus, over this period of time the surface layers of the coupling surfaces 10,11 of the first portion 2 and of the second portion 3 are mutually welded, obtaining a fixing that is rigid, resistant and safe over time. As evincible from the previous description, locking the first portion 2 to the second portion 3 of the spacer device 1 according to the invention can be obtained in a quick, accurate, simple and inexpensive manner, without using specific mechanical locking means or other expensive or complex systems.

Furthermore, it should be observed that both the first portion 2 and the second portion 3 of the spacer device according to the invention are entirely without exposed metal parts: actually, the surfaces thereof are completely coated with biologically compatible material, eliminating the possibility of infections occurring.

Furthermore, there can also be provided other examples of the locking method described above, for example providing for other equivalent methods for distributing the bonding component on the coupling surfaces 10, 11. For example, in some examples of the method the bonding component can be applied on both coupling surfaces 10, 11 instead of only one.

Another embodiment of the spacer device according to the invention is illustrated in figures 14,15.

This embodiment differs from the previous one due to the fact that the positions of the coupling surfaces 10,11 of the first portion 2 and of the second portion 3 are inverted.

More in detail, in this embodiment the screw 10 is provided in the second portion 3, i.e. in the spherical head 8, while the nut screw 11 is provided in the first portion 2, i.e. in the end 7 of the stem 5.

As observed in particular in figure 14, the screw 10 is provided within a cylindrical seat 13 provided in the spherical head 8.

In order to confer the required mechanical resistance, the screw 10 is provided with a respective second core 14 incorporated in the head 8.

The core 14 can for example be made of metal material, or any other material having suitable characteristics. The first portion 2, as described in the previous embodiment comprises a first reinforcement core 15, for example made of metal material or any other material having suitable characteristics.

The first portion 2 comprises, in the present embodiment, an end shank 16 of the stem 5, having a smooth cylindrical outer surface.

The outer diameter of the end shank 16 is such to be inserted in the cylindrical seat 13 with considerable accuracy, without requiring applying force.

The nut screw 11 is obtained, in particular, in a bushing 17 inserted in a respective housing 18 provided in the first core 15 of the first portion 2.

The bushing 17 is made of the same biologically compatible material with which the remaining parts of the first portion 2 are made.

As observable in the section of the figure 15, the first core 15 of the first portion 2 is entirely covered by the biologically compatible material.

Thus, there are no exposed metal parts also in this embodiment.

Also in this embodiment the biologically compatible material comprises polymethylmethacrylate.

In the present embodiment, the visual indicator 12 comprises a band made along the outer surface of the shank 16 of the stem 5, as illustrated in figure 14. The band of the visual indicator 12 may be obtained according to the criteria described regarding the preceding embodiment.

The second portion 3 is locked to the first portion 2 in a manner entirely similar to the one described regarding the preceding embodiment.

In particular, the stage of distributing a predefined amount of bonding component on at least one of the coupling surfaces 10,11 occurs by filling the nut screw 11 provided in the first portion 2, and then applying a cap thereon.

Then, there follows the stages described regarding the preceding embodiment, obtaining the same results and the same advantages described previously.

The present invention thus conceived can be subjected to various modifications and variants all falling within the scope of protection of the claims.

## Claims

1. Spacer device (1) for the two-stage treatment of infections of the prosthesis of the human limbs, i.e. for a treatment including a first stage of removing an infected arthroprosthesis and a second stage of a new re-implant of arthroprosthesis upon clearing the tissues of the patient in terms of infection, said spacer being made of biologically compatible material comprising polymethylmethacrylate adapted to be added and/or which can be added with pharmaceutical products, active and/or therapeutic ingredients, comprising a first portion (2) adapted to be fixed to a corresponding bone bed, a second portion (3) adapted to be inserted in a corresponding articular area of the patient, said first portion (2) and said second portion (3) being connected through adjustable joining means (4), wherein said joining means (4) comprise mutual coupling surfaces (10,11) respectively provided in said first portion (2) and said second portion (3), wherein said mutual coupling surfaces (10,11) comprise a screw (10) and a nut screw (11), wherein said screw (10) is provided in said first portion (2) and said nut screw (11) is provided in said second portion (3) or wherein said screw (10) is provided in said second portion (3) and said nut screw (11) is provided in said first portion (2), **characterised in that** said mutual coupling surfaces (10,11) are coated with said biologically compatible material which comprises polymethylmethacrylate **and in that** said screw (10) and nut screw (11) are of the type having a threading with rounded profile.

2. Device according to claim 1, wherein said first portion (2) comprises a stem (5) to be inserted in the proximal part of a femur, and/or wherein said stem (5) has different lengths and sections so as to be adapted to the different sizes of the femurs of the patients and/or wherein said stem (5) is substantially frusto-conical so as to hold the trochanter of the femur on which said stem (5) should be implanted.

3. Device according to claim 1 or 2, wherein said second portion (3) comprises a substantially spherical head (8), said head being provided in different dimensions, in particular with different diameter of the sphere so as to be adapted to the different sizes of the articular capsules of the patients.

4. Device according to claim 2, wherein said stem (5) comprises an end shank (16) in which said nut screw (11) is provided.

5. Device according to any one of the preceding claims, wherein said first portion (2) comprises a visual indicator (12) of the position of complete unfastening of said second portion (3).

6. Device according to claim 4, wherein said screw (10) is provided within a cylindrical seat (13) provided in said spherical head (8) and/or wherein said screw (10) is provided with a respective second core (14) incorporated in said head (8).

7. Device according to any one of claims 4 or 6, wherein said stem (5) is provided with a first core (15) and wherein said nut screw (11) is obtained with a bushing (17) inserted in a respective housing (18) provided in said first core (15).

8. Device according to any one of the preceding claims, comprising locking means adapted to fix the position of said adjustable joining means (4) and thus the mutual position between said first and second portion.

9. Device according to any one of the preceding claims, wherein said locking means comprise portions of said mutual coupling surfaces (10, 11) rigidly welded by applying a bonding component and/or wherein said bonding component comprises acrylic bone cement, or a cyanoacrylate bonding agent, or an organic solvent or a mixture of several components, such as chloroform, methyl methacrylate, ethyl acetate, dichloromethane, etcetera.

10. Device according to claims 5, wherein said visual indicator (12) comprises a band made along the outer surface of said screw (10) or wherein said visual indicator (12) comprises a band made along the outer surface of said end shank (16).

11. Device according to any one of the preceding claims, wherein said biologically compatible material comprises one or more first pharmaceutical products, active and/or therapeutic ingredients adapted to be released in the tissues of the patient adjacent to the device or wherein said biologically compatible material is without pharmaceutical products, active and/or therapeutic ingredients and/or wherein said biologically compatible material is porous and can be added, for example by impregnation, with one or more pharmaceutical products active and/or therapeutic ingredients identical or different with respect to the first pharmaceutical products, active and/or therapeutic ingredients.

12. Device according to any one of the preceding claims, wherein said pharmaceutical products, active and/or therapeutic ingredients comprise one or more of the following products: antibiotics, for example gentamicin, vancomycin, antiseptics, antimycotics, chemotherapeutic substances, etcetera, or other active ingredients.

## Patentansprüche

1. Abstandshalter (1) für die zweistufige Behandlung von Infektionen der Prothese der menschlichen Gliedmaßen, d.h. für eine Behandlung einschließlich einer ersten Stufe des Entfernens einer infizierten Arthroprothese und einer zweite Stufe eines neuen Reimplantierens der Arthroprothese nach Beseitigung des Gewebes des Patienten in Hinblick auf Infektionen, wobei der besagte Abstandshalter aus biologisch kompatiblem Material, umfassend Polymethylmethacrylat, hergestellt ist, das geeignet ist, hinzugefügt zu werden und/oder mit pharmazeutischen Produkten, aktiven und/oder therapeutischen Bestandteilen ergänzt werden kann, umfassend einen ersten Abschnitt (2), der geeignet ist, an einem entsprechenden Knochenbett fixiert zu werden, einen zweiten Abschnitt (3), der geeignet ist, in einen entsprechenden Gelenkbereich des Patienten eingeführt zu werden, wobei der besagte erste Abschnitt (2) und der besagte zweite Abschnitt (3) durch einstellbare Verbindungsmittel (4) verbunden sind, worin die besagten Verbindungsmittel (4) gegenseitige Kupplungsflächen (10, 11) umfassen, die jeweils in dem besagten ersten Abschnitt (2) und dem besagten zweiten Abschnitt (3) vorgesehen sind, worin die besagten gegenseitigen Kupplungsflächen (10, 11) eine Schraube (10) und eine Mutterschraube (11) umfassen, worin die besagte Schraube (10) in dem besagten ersten Abschnitt (2) vorgesehen ist und die besagte Mutterschraube (11) in dem besagten zweiten Abschnitt (3) vorgesehen ist oder worin die besagte Schraube (10) in dem besagten zweiten Abschnitt (3) vorgesehen ist und die besagte Mutterschraube (11) in dem besagten ersten Abschnitt (2) vorgesehen ist, **dadurch gekennzeichnet, dass** die besagten gegenseitigen Kupplungsflächen (10, 11) mit dem besagten biologisch kompatiblen Material beschichtet sind, das Polymethylmethacrylat umfasst, und dadurch, dass die besagte Schraube (10) und Mutterschraube (11) von dem Typ sind, die ein Gewinde mit abgerundetem Profil aufweisen.

2. Vorrichtung nach Anspruch 1, worin der erste besagte Abschnitt (2) einen Schaft (5) umfasst, der in den proximalen Teil eines Femurs einzuführen ist, und/oder worin der besagte Schaft (5) unterschiedliche Längen und Querschnitte aufweist, um an die unterschiedlichen Größen der Femurs der Patienten angepasst zu werden, und/oder worin der besagte Schaft (5) im Wesentlichen kegelstumpfförmig ist, um den Trochanter des Femurs, auf dem der besagte Schaft (5) implantiert werden soll, zu halten.

3. Vorrichtung nach Anspruch 1 oder 2, worin der besagte zweite Teil (3) einen im Wesentlichen kugelförmigen Kopf (8) umfasst, wobei der besagte Kopf in verschiedenen Abmessungen, insbesondere mit unterschiedlichem Durchmesser der Kugel, vorgesehen ist, um an die verschiedenen Größen der Gelenkkapseln der Patienten angepasst zu werden.

4. Vorrichtung (1) nach Anspruch 2, worin der besagte Schaft(5) einen Endschenkel (16), in dem die besagte Mutterschraube (11) vorgesehen ist, umfasst.

5. Vorrichtung nach irgendeinem der vorangegangenen Ansprüche, worin der besagte erste Teil (2) einen optischen Anzeiger (12) der Position des vollständigen Lösens des besagten zweiten Abschnitts (3) umfasst.

6. Vorrichtung nach Anspruch 4, worin die besagte Schraube (10) innerhalb eines zylindrischen Sitzes (13) vorgesehen ist, der in dem besagten kugelförmigen Kopf (8) vorgesehen ist, und/oder worin die besagte Schraube (10) mit einem jeweiligen zweiten Kern (14) versehen ist, der in dem besagten Kopf (8) integriert ist.

7. Vorrichtung nach irgendeinem der Ansprüche 4 oder 6, worin der besagte Schaft (5) mit einem ersten Kern (15) versehen ist und worin die besagte Mutterschraube (11) mit einer Buchse (17) erhalten wird, die in ein jeweiliges Gehäuse (18) eingesetzt ist, das in dem besagten ersten Kern (15) vorgesehen ist.

8. Vorrichtung nach irgendeinem der vorangegangenen Ansprüche, umfassend Verriegelungsmittel, die geeignet sind, die Position der besagten einstellbaren Verbindungsmittel (4) und somit die gegenseitige Position zwischen dem besagten ersten und zweiten Abschnitt zu fixieren.

9. Vorrichtung nach irgendeinem der vorangegangenen Ansprüche, worin die besagten Verriegelungsmittel Abschnitte der besagten gegenseitigen Kupplungsflächen (10, 11) umfassen, die durch Aufbringen einer Bindekomponente starr verschweißt sind und/oder worin die besagte Bindekomponente acrylischen Knochenzement oder ein Cyanacrylat-Bindemittel oder ein organisches Lösungsmittel oder eine Mischung mehrerer Komponenten, wie Chloroform, Methylmethacrylat, Ethylacetat, Dichlormethan usw. umfasst.

10. Vorrichtung nach Anspruch 5, worin der besagte optische Anzeiger (12) ein Band umfasst, das entlang der Außenfläche der besagten Schraube (10) hergestellt ist, oder worin der besagte optische Anzeiger (12) ein Band umfasst, das entlang der Außenfläche des besagten Endschenkels (16) hergestellt ist.

11. Vorrichtung nach irgendeinem der vorangegangenen Ansprüche, worin das besagte biologisch kompatible Material ein oder mehrere erste pharmazeutische Produkte, aktive und/oder therapeutische Bestandteile umfasst, die geeignet sind, in die an die Vorrichtung angrenzenden Gewebe des Patienten freigesetzt zu werden, oder worin das besagte biologisch kompatible Material ohne pharmazeutische Produkte, aktive und/oder therapeutische Bestandteile ist und/oder worin das besagte biologisch kompatible Material porös ist und, beispielsweise durch Imprägnierung, mit einem oder mehreren pharmazeutischen Produkten, aktiven und/oder therapeutischen Bestandteilen, die identisch oder verschieden in Bezug auf die ersten pharmazeutischen Produkte, aktiven und/oder therapeutischen Bestandteile sind, ergänzt werden kann.

12. Vorrichtung nach irgendeinem der vorangegangenen Ansprüche, worin die besagten pharmazeutischen Produkte, aktiven und/oder therapeutischen Bestandteile eines oder mehrere der folgenden Produkte umfassen: Antibiotika, zum Beispiel Gentamicin, Vancomycin, Antiseptika, Antimykotika, chemotherapeutische Substanzen, usw., oder andere aktive Bestandteile.

## Revendications

1. Dispositif d'entretoise (1) pour le traitement en deux phases des infections de la prothèse des membres du corps humain, c'est-à-dire pour un traitement comprenant un premier stade de dépose d'une arthroprosthèse infectée et un deuxième stade de nouvelle réimplantation d'arthroprosthèse après avoir dégagé l'infection des tissus du patient, ladite entretoise étant constituée de matière biocompatible comprenant du polyméthylméthacrylate adapté pour être ajouté et/ou qui peut être complété de produits pharmaceutiques, d'ingrédients actifs et/ou thérapeutiques, comprenant une première partie (2) adaptée pour être fixée à un lit osseux correspondant, une deuxième partie (3) adaptée pour être insérée dans une zone articulaire correspondante du patient, ladite première partie (2) et ladite deuxième partie (3) étant reliées par des moyens de jonction ajustables (4), dans lequel lesdits moyens de jonction (4) comprennent des surfaces d'accouplement mutuel (10, 11) disposées respectivement dans ladite première partie (2) et dans ladite deuxième partie (3), dans lequel lesdites surfaces d'accouplement mutuel (10, 11) comprennent une vis (10) et une vis à écrou (11), dans lequel ladite vis (10) est disposée dans ladite première partie (2) et ladite vis à écrou (11) est disposée dans ladite deuxième partie (3) ou dans lequel ladite vis (10) est disposée dans ladite deuxième partie (3) et ladite vis à écrou (11) est disposée dans ladite première partie (2), **caractérisé en ce que** lesdites surfaces d'accouplement mutuel (10, 11) sont revêtues de ladite matière biocompatible qui comprend du polyméthylméthacrylate et **en ce que** ladite vis (10) et ladite vis à écrou (11) sont du type ayant un filetage avec un profil arrondi.

2. Dispositif selon la revendication 1, dans lequel ladite première partie (2) comprend une tige (5) destinée à être insérée dans la partie proximale d'un fémur, et/ou dans lequel ladite tige (5) a différentes longueurs et sections de façon à être adaptée aux différentes tailles des fémurs des patients et/ou dans lequel ladite tige (5) est sensiblement tronconique pour maintenir le trochanter du fémur sur lequel ladite tige (5) devrait être implantée.

3. Dispositif selon la revendication 1 ou 2, dans lequel ladite deuxième partie (3) comprend une tête sensiblement sphérique (8), ladite tête étant fournie dans différentes dimensions, en particulier avec un diamètre différent de la sphère de façon à être adaptée aux différentes tailles des capsules articulaires des patients.

4. Dispositif selon la revendication 2, dans lequel ladite tige (5) comprend une tige d'extrémité (16) dans laquelle ladite vis à écrou (11) est disposée.

5. Dispositif selon l'une des revendications précédentes, dans lequel ladite première partie (2) comprend un indicateur visuel (12) de la position de détachement complet de ladite deuxième partie (3).

6. Dispositif selon la revendication 4, dans lequel ladite vis (10) est disposée dans un siège cylindrique (13) prévu dans ladite tête sphérique (8) et/ou dans lequel ladite vis (10) est dotée d'un deuxième noyau (14) respectif intégré dans ladite tête (8).

7. Dispositif selon l'une des revendications 4 ou 6, dans lequel ladite tige (5) est dotée d'un premier noyau (15) et dans lequel ladite vis à écrou (11) est obtenue avec une douille (17) insérée dans un logement (18) respectif disposé dans ledit premier noyau (15).

8. Dispositif selon l'une des revendications précédentes, comprenant des moyens de verrouillage adaptés pour fixer la position desdits moyens de jonction (4) ajustables et donc la position mutuelle entre ladite première et ladite deuxième parties.

9. Dispositif selon l'une des revendications précédentes, dans lequel lesdits moyens de verrouillage comprennent des parties de ladite surfaces d'accouplement mutuel (10, 11) soudées rigidement en appliquant un composant de collage et/ou dans lequel ledit composant de collage comprend un ciment osseux acrylique, ou un agent de collage cyanoacrylate, ou un solvant organique ou un mélange de plusieurs composants, tels que le chloroforme, le méthacrylate de méthyle, l'acétate d'éthyle, le dichlorométhane, etc.

10. Dispositif selon la revendication 5, dans lequel ledit indicateur visuel (12) comprend une bande réalisée le long de la surface extérieure de ladite vis (10) ou dans lequel ledit indicateur visuel (12) comprend une bande réalisée le long de la surface extérieure de ladite tige d'extrémité (16).

11. Dispositif selon l'une des revendications précédentes, dans lequel ladite matière biocompatible comprend un ou plusieurs premiers produits pharmaceutiques, ingrédients actifs et/ou thérapeutiques adaptés pour être libérés dans les tissus du patient adjacents au dispositif ou dans lequel ladite matière biocompatible est exempte de produits pharmaceutiques, d'ingrédients actifs et/ou thérapeutiques et/ou dans lequel ladite matière biocompatible est poreuse et peut être complétée, par exemple par imprégnation, d'un ou de plusieurs produits pharmaceutiques, ingrédients actifs et/ou thérapeutiques identiques ou différents des premiers produits pharmaceutiques, ingrédients actifs et/ou thérapeutiques.

12. Dispositif selon l'une des revendications précédentes, dans lequel lesdits produits pharmaceutiques, ingrédients actifs et/ou thérapeutiques comprennent un ou plusieurs des produits suivants : antibiotiques, par exemple gentamicine, vancomycine, antiseptiques, antifongiques, substances chimiothérapeutiques, etc., ou autres ingrédients actifs.
